# EUROPEAN PATENT SPECIFICATION

(11) **EP 1 539 050 B1**
(45) Date of publication and mention of the grant of the patent: **01.08.2007**
(21) Application number: 03797472.2
(22) Date of filing: 19.09.2003
(51) Int. Cl.: A61F 2/42

(54) **ARTHROPLASTY IMPLANT**
ARTHROPLASTIE-IMPLANTAT
IMPLANT ARTHROPLASTIQUE

(30) Priority: 19.09.2002 ZA 200207516
(43) Date of publication of application: 15.06.2005
(73) Proprietor: De Villiers, Malan, 1675 Irene (ZA)
(72) Inventor: De Villiers, Malan, 1675 Irene (ZA)
(74) Representative: Johnstone, Helen Margaret
(86) International application number: PCT/IB2003/004051
(87) International publication number: WO 2004/026185

(56) References cited:
- WO-A-00/59411
- WO-A-01/03613
- WO-A-93/00053
- WO-A-02/089701
- DE-U- 29 514 169
- FR-A- 2 730 159
- US-A- 4 685 919
- US-A- 5 674 297

## Description

### BACKGROUND TO THE INVENTION

THIS invention relates to an arthroplasty implant.

The invention is particularly concerned with arthroplasty implants of the wrist and small bones of the hand and foot, such as metatarsophalangeal (MTP) joint implants, metacarpophalangeal (MCP) joint implants and proximal interphalangeal (PIP) joint implants.

Various types of implants for such joints have been proposed and are in use. It is however believed that the known implants, most of which are of two part construction, suffer from one disadvantage or other that either limits their flexibility, load-transmitting ability or life expectancy.

Document WO 01/03613 discloses an arthroplasty implant comprising three components.

Documents FR2730159 and WO02/089701, the latter belonging to the state of the art according to Article 54(3) EPC, disclose an arthroplasty implant with a first, a second and an intermediate component, the latter component being slidable with respect to the first and second components.

### SUMMARY OF THE INVENTION

According to the present invention there is provided an arthroplasty implant for providing a joint between first and second bones of a wrist, hand or foot, the implant comprising:
- a first component defining a concave surface and having first connection means for connecting it to the first bone;
- a second component defining a convex surface an having second connection means for connecting it to the second bone;
- an intermediate component for location between the first and second components and defining a convex surface which is slidable on the concave surface of the first component to allow articulation between the first component and the intermediate component and a concave surface slidable on the convex surface of the second component to allow articulation between the second component and the intermediate component, and
- means for preventing the intermediate component from separating laterally from at least one of the first and second components.

The concave surface of the first component and the convex surface of the intermediate component are preferably complementally, spherically curved. In the preferred embodiments, the convex surface of the second component and the concave surface of the intermediate component are defined by radii of curvature which differ in mutually orthogonal directions. The length of the convex surface in a direction defined by a relatively large radius of curvature is preferably greater than the length of that surface in a direction defined by a relatively small radius of curvature.

In all cases, the first and second components should be capable of translation and articulation relative to the intermediate component.

One embodiment of the invention comprises a central projection on the concave surface of the first component and a central opening in the convex surface of the intermediate component, the projection in use locating loosely in the opening to prevent lateral separation of the intermediate and first components.

In another embodiment of the invention one of the first component and the intermediate component includes a laterally outwardly facing projection and the other of the first component and the intermediate component includes a laterally inwardly facing recess, the projection in use interacting with the recess to prevent lateral separation of the intermediate and first components. Typically in this embodiment, the first component includes an annular wall bounding the concave surface of that component, the peripheral wall being formed with an annular undercut defining the laterally inwardly facing recess, and the intermediate component includes an annular rib defining the laterally outwardly facing projection, interaction in use between the rib and the undercut preventing lateral separation of the intermediate and first components.

Other features of the invention are defined in the appended claims.

### BRIEF DESCRIPTION OF THE DRAWINGS

The invention will now be described in more detail, by way of example only, with reference to the accompanying drawings in which:
- **Figure 1**: shows a perspective view of a first component of an arthroplasty implant according to a first embodiment of the invention;
- **Figure 2**: shows a side view of the component seen in Figure 1;
- **Figure 3**: shows a perspective view of a second component of an arthroplasty implant according to first embodiment of the invention;
- **Figure 4**: shows a side view of the component seen in Figure 3;
- **Figure 5**: shows a plan view of the component seen in Figure 3;
- **Figure 6**: shows a perspective view of an intermediate component of an arthroplasty implant according to the first embodiment of the invention;
- **Figure 7**: shows a plan view of the intermediate component seen in Figure 6;
- **Figure 8**: shows a cross-section at the line 8-8 in Figure 7;
- **Figure 9**: shows a cross-section at the line 9-9 in Figure 7;
- **Figure 10**: shows a side view of an assembled arthroplasty implant according to the first embodiment of the invention with the first and intermediate components in a neutral position before articulation between them;
- **Figure 11**: shows a similar side view of the assembled arthroplasty implant seen in Figure 10 after maximum articulation between the first and intermediate components;
- **Figure 12**: shows a plan view of the assembled arthroplasty implant seen in Figure 10 after maximum articulation between the first and intermediate components.
- **Figure 13**: shows a perspective view of the first component of an arthroplasty implant according to a second embodiment of the invention;
- **Figure 14**: shows a side view of the first component seen in Figure 13;
- **Figure 15**: shows a perspective view of the intermediate component of an arthroplasty implant according to the second embodiment of the invention;
- **Figure 16**: shows a side view in the direction of the arrow 16 of the intermediate component seen in Figure 15;
- **Figure 17**: shows a side view in the direction of the arrow 17 of the intermediate component seen in Figure 15;
- **Figure 18**: shows a side view of an assembled arthroplasty implant according to the second embodiment of the invention with the first and intermediate components in a neutral position before articulation between them; and
- **Figure 19**: shows a similar side view of the assembled arthroplasty implant seen in Figure 18 after maximum articulation between the first and intermediate components.

### DESCRIPTION OF PREFERRED EMBODIMENTS

The drawings illustrate individual components of preferred metatarsophalangeal (MTP) joint implants, and the assembled MTP implants. In each case the implant consists of three individual components.

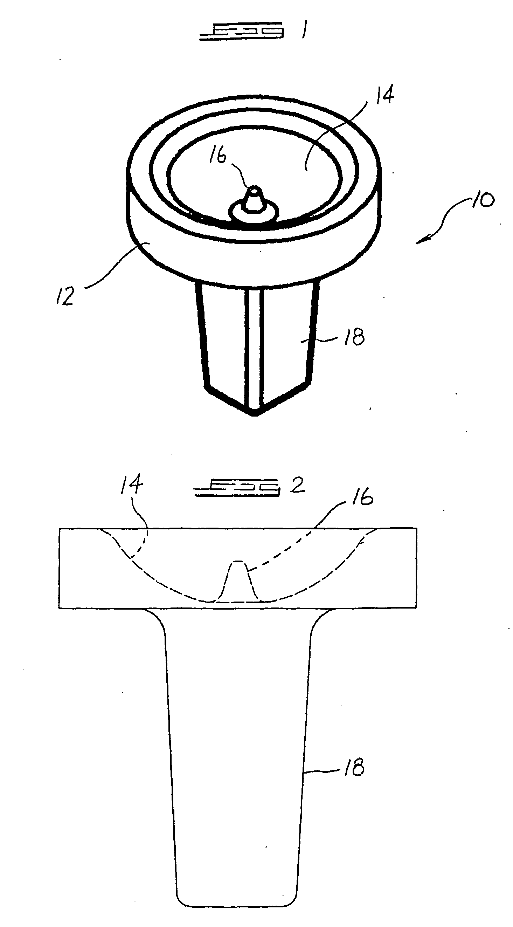

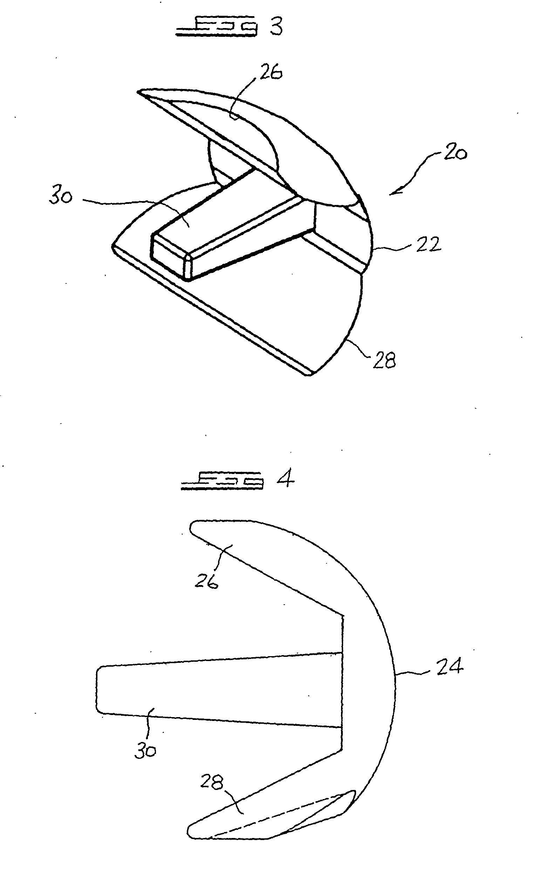

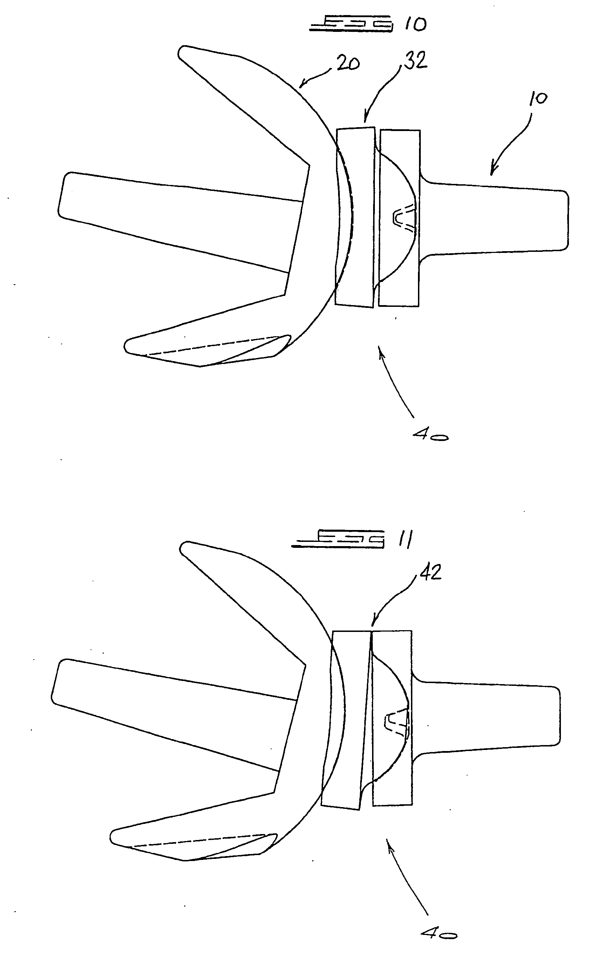

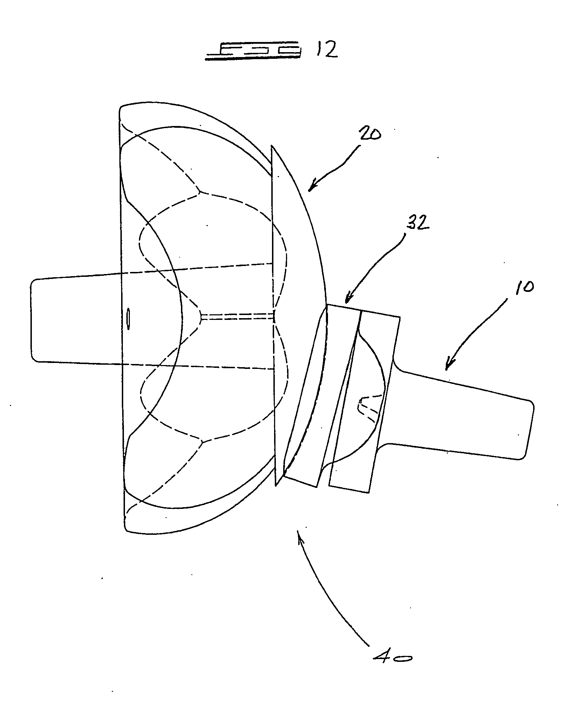

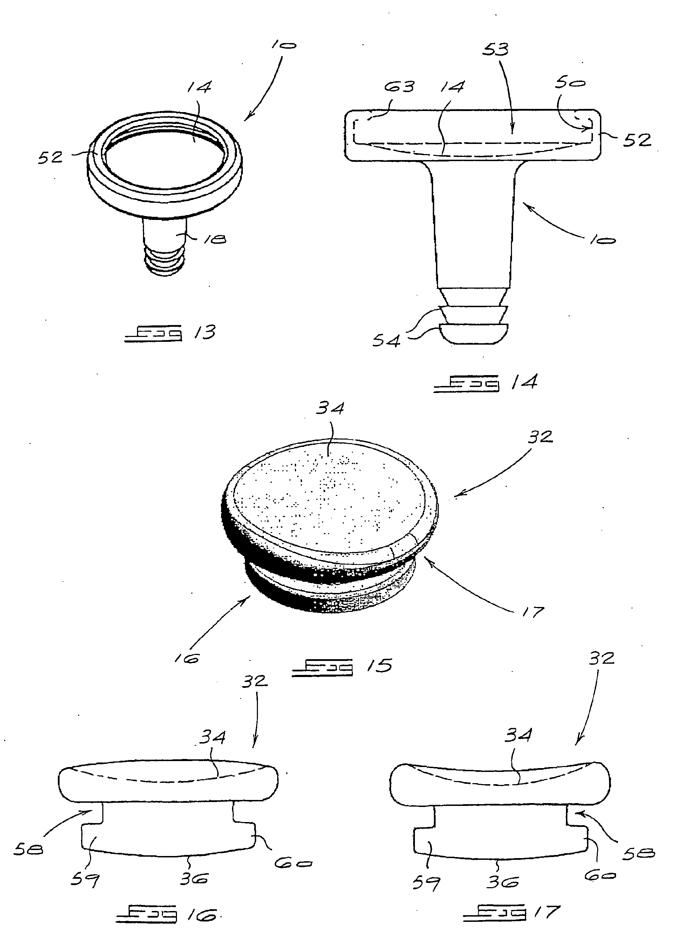

A first embodiment of the invention is illustrated in Figures 1 to 12 of the drawings. In this embodiment, Figures 1 and 2 illustrate a first, phalangeal component 10 which is connected in use to a phalanx. It includes a body 12 formed with a spherically curved, concave surface 14. Projecting centrally from the surface 14 is a conical peg 16 and projecting rearwardly from the body 12 is a tapered post 18 of square cross-section. In use, the post 18 is placed and anchored in a predrilled hole in the phalanx.

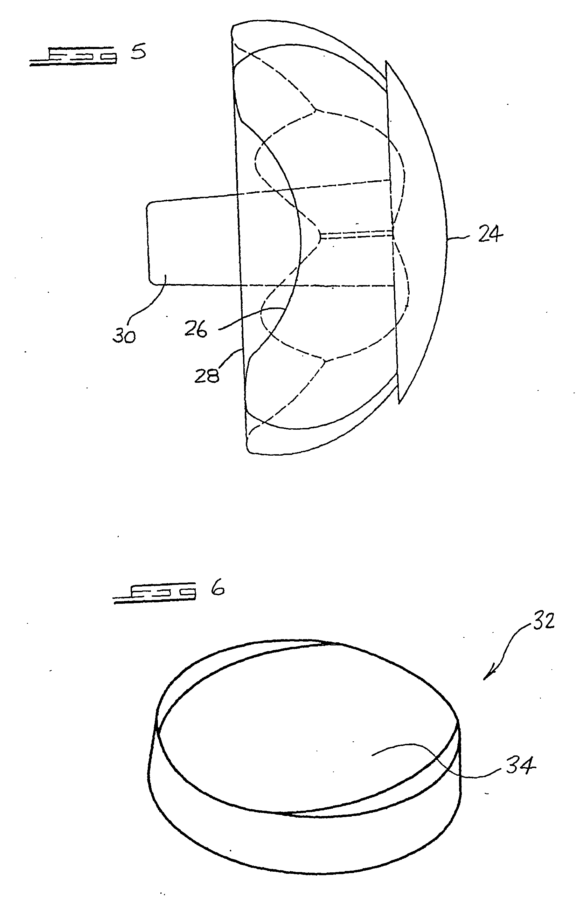

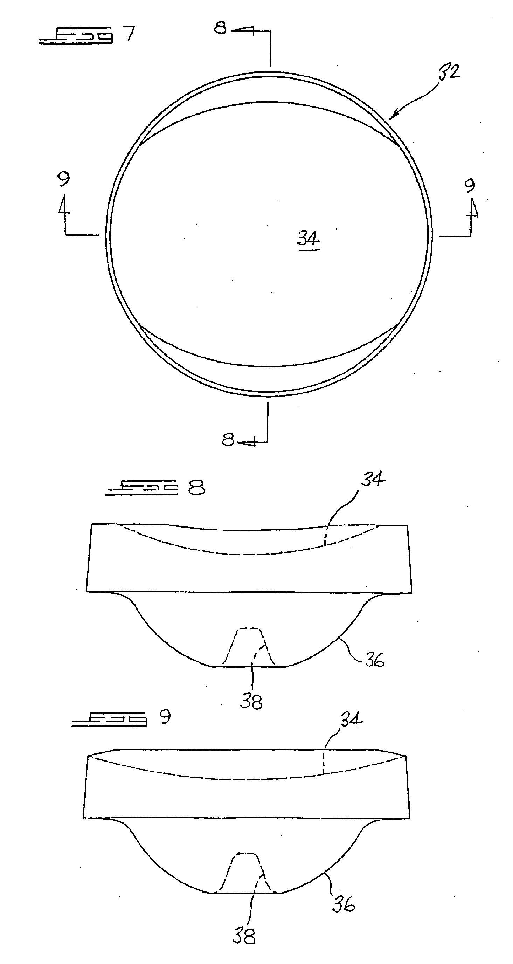

Figures 3 to 5 illustrate a second, tarsal component 20 which is connected in use to the associated tarsus. It includes a body 22 with a convexly curved surface 24 and curved skirts 26, 28. The radius of curvature of the surface 24 in the view of Figure 4 is less than the radius of curvature in the view of Figure 5. Projecting rearwardly from the body 22 is a tapered post 30 of square cross-section. In use the post 30 is placed and anchored in a predrilled hole in the tarsus.

Both the phalangeal component 10 and the tarsal component 20 are made in one piece of grade 5 titanium, their curved surfaces 14 and 24 being provided with a titanium nitride finish.

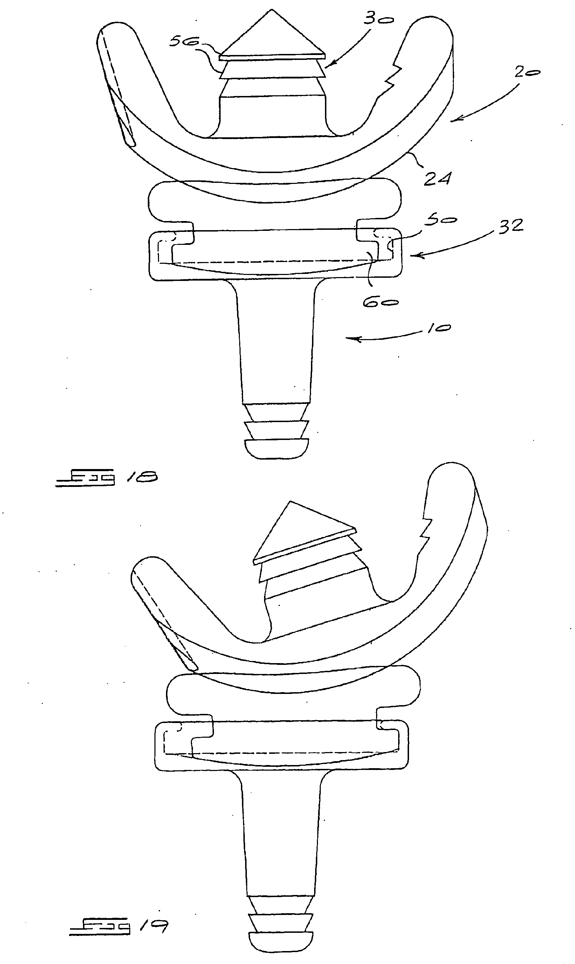

Figures 6 to 9 illustrate an intermediate component in the form of a meniscus 32 which is located in the assembled MTP implant between the phalangeal and tarsal components 10 and 20. The meniscus 32 is made of a low friction plastic material, in this case an ultra high molecular weight polyethylene (UHMWPE) available under the name ORTHOSOL^{™}. One side of the meniscus is formed with a concave surface 34 and the opposite side with a convex surface 36. The convex surface is spherically curved and is formed centrally with a conical recess or socket 38. The concave surface 34 is not spherical. The radius of curvature of the surface 34 in Figure 8, which matches radius of curvature of the surface 24 in Figure 4, is less than the radius of curvature of the surface 34 in Figure 9, which matches the radius of curvature of the surface 24 in Figure 5. It will accordingly be understood that the concave surface 34 of the meniscus is complemental to the convex surface 24 of the tarsal component 20, and that the convex surface 36 of the meniscus is complemental to the concave surface 14 of the phalangeal component 10.

Figures 10 to 12 illustrate an assembled MTP arthroplasty implant 40, according to the first embodiment of the invention, and consisting of the three components 10, 20 and 32. The meniscus 32 is located between the phalangeal and tarsal components 10 and 20 with the various concave and convex surfaces in cooperating relationship with one another. The peg 16 of the phalangeal component is located in the socket 38 of the meniscus 32. In this regard it will be noted that the transverse dimension of the peg is somewhat less than the transverse dimension of the socket at any given point along the length of the peg and socket.

In Figure 10, the phalangeal component 10 and the meniscus 32 are at a neutral orientation with one another, i.e. they are axially aligned and no articulation or translation has taken place between them. Figure 11 illustrates the situation after maximum permitted articulation and translation has taken place between these components. It will be noted that in Figure 11, edge regions of the phalangeal component 10 and meniscus 32 come into contact with one another, as indicated by the arrow 42. Further articulation in the same sense past this condition is impossible. The fact that the socket 38 is oversize with respect to the peg 16 permits translation and maximum articulation to take place, but it will be noted that in Figure 11 the peg 16 also abuts the side of the socket 38 to prevent further articulation or translation.

Throughout the permitted range of movement between the phalangeal component and the meniscus, the peg 16 remains located in the socket 38. This prevents the meniscus from separating laterally from the phalangeal component, i.e. holds the meniscus captive relative to the phalangeal component at all times.

In Figures 10 and 11, there is no change in the positional relationship of the meniscus and the tarsal component 20. Given the complemental curvature of their respective convex and concave surfaces, it will however be understood that these components are free to slide over one another and, in doing so, to articulate relative to one another. This is illustrated by Figure 12, which shows the meniscus, and with it the phalangeal component, after relative sliding, i.e. translation, and articulation has taken place. The convex surface 24 of the tarsal component 20 is substantially larger than the complemental concave surface 34 of the meniscus, allowing translation and articulation to take place over a wide range of positions and angles.

A second embodiment of the invention is illustrated in Figures 13 to 19 of the drawings. In these Figures features corresponding to features seen in Figures 1 to 12 are indicated by the same reference numerals.

Notable differences between the first or phalangeal component 10 illustrated in Figures 13 and 14 and the first component 10 seen in Figures 1 and 2 are the absence of the central peg 16 and the inclusion of an annular, peripheral wall 52 which bounds the concave surface 14 and which is formed with an undercut 50 defining a laterally inwardly facing recess. The wall 52 and concave surface in combination define a cup-shaped receptacle 53.

Another difference between the component 10 of Figures 13 and 14 and that of Figures 1 and 2 is the face that the post 18 has a round cross-section and is provided at its end with barb formations 54.

The second or tarsal component 20 of the second embodiment is seen in Figures 18 and 19. This component differs from the second component illustrated in Figures 3 to 5 in that the post 30 is of round cross-section and carries barb formations 56 at its end. The structure defining the convex surface 24 is also slightly different, as illustrated.

It is believed that the barb formations 54 and 56 will be able to provide better anchorage of the posts 18 and 30 in their respective predrilled holes in the phalanx and tarsus respectively.

The intermediate component or meniscus 32 of the second embodiment is illustrated in Figures 15 to 17. As will be apparent from Figures 16 and 17, the concave surface 34 of this meniscus is similar to that of the first embodiment. The convex surface 36 of the second embodiment is however defined by a somewhat greater radius of curvature than the corresponding surface in the first embodiment. Anther difference between the convex surface 36 of the second embodiment and that of the first embodiment is the absence of the central recess or socket 38. The meniscus also cinludes an annular groove 58, with a portion 59 of the meniscus beneath this groove presenting an annular, outwardly facing rib 60.

Figures 18 and 19 illustrated the second embodiment in an assembled condition. The portion 59 of the meniscus is received in the cup-shaped receptacle 53. Typically the outer diameter of the rib 60 will be such that the portion 59 is either be a very close fit or a press fit through the opening defined by the inner rim 63 of the undercut side wall 52. A comparison of Figures 18 and 19 shows how the first component and meniscus can both articulate and translate laterally relative to one another. It will however be understood that throughout the range of permitted translation and articulation, the lower portion 59 of the meniscus is held captive relative to the first component 10 by the undercut side wall 52. Thus in this embodiment the interaction of the rib 60 and the undercut recess 50 prevents the components from separating laterally from one another.

A comparison of Figures 18 and 19 also illustrates the ability of the implant to accommodate a wide range of translation and articulation between the second component 20 and the meniscus.

Referring again to the first embodiment, a comparison of Figures 10 and 11 on one hand and Figure 12 on the other hand indicates that articulation and translation between the components can take place in mutually orthogonal directions. The range of translation and articulated movement between the tarsal component 20 and the meniscus in one direction, illustrated by Figures 10 and 11, is greater than the corresponding range of movement in the orthogonal direction, illustrated by Figure 12. This feature is attributable to the shape of the structure defining the convex surface 24 and is provided for the reason that most small joints of the hand or foot are designed to flex primarily in one direction. Considering, for instance, a toe joint, the primary flexural movement of the toe is towards or away from the foot rather than at right angles thereto, although at least a small degree of movement in the latter sense must also be accommodated.

Figures 18 and 19 only illustrate the primary flexural movement, but it will be understood that a similar range of movement in the orthogonal direction is also possible in this embodiment.

In both embodiments the concave surface 34 of the meniscus and the convex surface of the tarsal component 20 is defined by radii of curvature which differ from one another in mutually orthogonal direction. This feature also contributes to movement in the primary direction. In the case of the first embodiment compare, for instance, Figures 4 and 5 illustrating the tarsal component and Figures 8 and 9 illustrating the meniscus. ln the case of the second component, a similar comparison may be made between Figures 16 and 17 illustrating the meniscus. In each case, it will be understood that sliding movement is easier in the direction defined by the larger radius of curvature, i.e. the more gentle curvature.

It is believed that the three component implants described above and illustrated in the drawings will provide for substantial flexibility in the implanted arthroplasty. Also, the relatively large bearing areas between the respective components will, it is believed, provide the arthroplasty with substantial longevity. Referring in particular to the phalangeal components 10 and the menisci 32, the fact that these components are retained in their cooperating relationship either by the interaction of the peg 16 and socket 38 or by the interaction of the portion 60 and the undercut 50 means that there still remains a large bearing area between the components to transmit generally axial loading.

Many modifications are possible within the scope of the invention. For instance, although it is considered beneficial in the first embodiment for the peg to abut the side of the socket, as illustrated in Figure 11, this is not critical to the performance of the implant. In the second embodiment described above, it is the interaction of an outwardly facing projection, i.e. the rib 60 and the inwardly facing recess, i.e, the undercut 50, which prevents lateral separation of the phalangeal component and meniscus. The rib and recess are included in the phalangeal component and meniscus respectively. It will however be understood that the situation could equally well be reversed, with a projection on the phalangeal component interacting with a recess on the intermediate member. In similar fashion the peg and recess arrangement of the first embodiment could be reversed with the peg on the intermediate component and the recess in the first or phalangeal component. Still further the concave and convex surfaces could be reversed so that, for instance, the palangeal component has the convex surface and the tarsal component has the concave surface, such surfaces interacting with a concave and convex surfaces respectively on the intermediate member. It is also feasible for means to be provided, eg in the form of the described peg and recess combination or the described rib and recess combination, to prevent separation of the intermediate member from both of the first and second components. It is the intention that all such variations are included with the scope of the invention.

Further, while specific mention has been made of MTP arthroplasty implants, it will be understood that the principles of the invention are equally applicable to other arthroplasty implants, including those mentioned at the outset, typically for the wrist or small bones of the hand or foot.

## Claims

1. An arthroplasty implant for providing a joint between first and second bones of a wrist, hand or foot, the implant comprising:
- a first component (10) defining a concave surface (14) and having first connection means (18) for connecting it to the first bone ;
- a second component (20) defining a convex surface (24) and having second connection means (30) for connecting it to the second bone ;
- an intermediate component (32) for location between the first and second components and defining a convex surface (36) which is slidable on the concave surface of the first component to allow articulation between the first component and the intermediate component and a concave surface (34) slidable on the convex surface of the second component to allow articulation between the second component and the intermediate component, and
- means (16,38) for preventing the intermediate component from separating laterally from at least one of the first and second components.

2. An implant according to claim 1 wherein the concave surface (14) of the first component (10) and the convex surface (36) of the intermediate component (32) are complementally, spherically curved.

3. An implant according to either one of the preceding claims wherein the convex surface (24) of the second component (20) and the concave surface (34) of the intermediate component (32) are defined by radii of curvature which differ in mutually orthogonal directions.

4. An implant according to any one of the preceding claims wherein the length of the convex surface (24) of the second component (20) in a direction defined by a relatively large radius of curvature is greater than the length of that surface in a direction defined by a relatively small radius of curvature.

5. An implant according to any one of the preceding claims wherein the first and second components (10, 20) are capable of translation and articulation relative to the intermediate component (32).

6. An implant according to any one of the preceding claims wherein the first and second components (10, 20) are made of grade 5 titanium and their respective concave and convex surfaces (14, 24) are provided with a titanium nitride finish.

7. An implant according to any one of the preceding claims wherein the intermediate component (32) is made of a low friction plastics material.

8. An implant according to claim 7 wherein the plastics material is ultra high molecular weight polyethylene.

9. An implant according to any one of the preceding claims wherein the first and second connection means (18, 30) comprise projecting posts locatable in holes in the respective bones.

10. An implant according to any one of the preceding claims and comprising a central projection (16) on the concave surface (14) of the first component (10) and a central opening (38) in the convex surface (36) of the intermediate component (32) the projection in use locating loosely in the opening to prevent lateral separation of the intermediate and first components.

11. An implant according to claim 10 wherein the concave surface (14) of the first component (10) and the convex surface (36) of the intermediate component (32) are bounded by peripheral edges which contact one another when relative movement between the first component and the intermediate component reaches a predetermined maximum limit.

12. An implant according to any one of claims 1 to 9 wherein one of the first component (10) and the intermediate component (32) includes a laterally outwardly facing projection (60) and the other of the first component and the intermediate component includes a laterally inwardly facing recess (50), the projection in use interacting with the recess to prevent lateral separation of the intermediate and first components.

13. An implant according to claim 12 wherein the first component (10) includes an annular wall (52) bounding the concave surface (14) of that component, the wall being formed with an annular undercut (50) defining the laterally inwardly facing recess, and the intermediate component (32) includes an annular rib (60) defining the laterally outwardly facing projection, interaction in use between the rib and the undercut preventing lateral separation of the intermediate and first components.

14. An implant according to any one of the preceding claims wherein the first component (10) is a phalangeal component of a metatarsophalangeal joint implant and is connectable to a phalanx, and the second component (20) is a tarsal component of the joint and is connectable to a tarsus.

## Patentansprüche

1. Ein Gelenkimplantat zum Herstellen einer Gelenkverbindung zwischen einem ersten und einem zweiten Knochen eines Handgelenks, einer Hand oder eines Fußes, umfassend:
- eine erste Komponente (10), die eine konkave Oberfläche (14) aufweist und ein erstes Verbindungsmittel (18) zum Verbinden mit dem ersten Knochen hat;
- eine zweite Komponente (20), die eine konvexe Oberfläche (24) aufweist, und ein zweites Verbindungsmittel (30) zum Verbinden mit dem zweiten Knochen hat;
- eine mittlere Komponente (32), die zwischen der ersten und zweiten Komponente angeordnet wird und eine konvexe Oberfläche (36) aufweist, die auf der konkaven Oberfläche der ersten Komponente verschiebbar ist, um ein Abwinkeln zwischen der ersten Komponente gegenüber der mittleren Komponente zu ermöglichen, und eine konkave Oberfläche (34), die auf der konvexen Oberfläche der zweiten Komponente verschiebbar ist, um eine Abwinkeln zwischen der zweiten Komponente gegenüber der mittleren Komponente zu ermöglichen,
- Mittel (16, 38) zum Verhindern, dass sich die mittlere Komponente von der ersten oder zweiten Komponente in lateraler Richtung abtrennt.

2. Implantat nach Anspruch 1, wobei die konkave Oberfläche (14) der ersten Komponente (10) und die konvexe Oberfläche (36) der mittleren Komponente (32) komplementär sphärisch gekrümmt sind.

3. Implantat nach einem der vorhergehenden Ansprüche, wobei die konvexe Oberfläche (24) zu der zweiten Komponente (20) und die konkave Oberfläche (34) der mittleren Komponente (32) Krümmungsradien aufweisen, die sich in gegenseitig orthogonalen Richtungen unterscheiden.

4. Implantat nach einem der vorhergehenden Ansprüche, wobei die Länge der konvexen Oberfläche (24) der zweiten Komponente (20) in einer Richtung, die durch einen relativ großen Krümmungsradius definiert ist, größer ist als die Länge dieser Oberfläche in einer Richtung, die durch einen relativ kleinen Krümmungsradius definiert ist.

5. Implantat nach einem der vorhergehenden Ansprüche, wobei die erste und zweite Komponente (10, 20) relativ zur mittleren Komponente (32) verschiebbar und abwinkelbar sind.

6. Implantat nach einem der vorhergehenden Ansprüche, wobei die erste und zweite Komponente (10, 20) aus 5-gradigem Titanium hergestellt und ihre jeweiligen konkaven und konvexen Oberflächen (14, 24) mit einer Titaniumnitrid-Oberfläche versehen sind.

7. Implantat nach einem der vorhergehenden Ansprüche, wobei die mittlere Komponente (32) aus einem Kunststoffmaterial mit geringerer Reibung hergestellt ist.

8. Implantat nach Anspruch 7, wobei das Kunststoffmaterial ein Polyethylen mit ultra hohem Molekulargewicht ist.

9. Implantat nach einem der vorhergehenden Ansprüche, wobei das erste und zweite Verbindungsmittel (18, 30) vorspringende Pfosten umfassen, die in Löchern in den jeweiligen Knochen platzierbar sind.

10. Implantat nach einem der vorhergehenden Ansprüche und umfassend einen zentralen Vorsprung (16) auf der konkaven Oberfläche (14) der ersten Komponente (10), und eine zentrale Öffnung (38) in der konvexen Oberfläche (36) der mittleren Komponente (32), wobei der Vorsprung während der Benutzung lose in der Öffnung angeordnet ist, um zu verhindern, dass sich die mittlere und erste Komponente voneinander trennen.

11. Implantat nach Anspruch 10, wobei die konkave Oberfläche (14) der ersten Komponente (10) und die konvexe Oberfläche (36) der mittleren Komponente (32) durch periphere Kanten begrenzt sind, die sich gegenseitig berühren, wenn die relative Bewegung zwischen der ersten Komponente und der mittleren Komponente ein vorgegebenes Maximum erreicht.

12. Implantat nach einem der Ansprüche 1 bis 9, wobei die erste Komponente (10) oder die mittlere Komponente (32) einen nach außen gerichteten Vorsprung (60), und die andere der ersten oder mittleren Komponente eine lateral nach innen gerichtete Ausnehmung (50) aufweist, wobei der Vorsprung bei Benutzung mit der Ausnehmung zusammen wirkt, um zu verhindern, dass sich die mittlere und erste Komponente in lateraler Richtung trennen.

13. Implantat nach Anspruch 12, wobei die erste Komponente (10) eine ringförmige Wand (52) umfasst, die die konkave Oberfläche (14) dieser Komponente begrenzt, wobei die Wand mit einer ringförmigen Hinterschneidung (15) ausgebildet ist, die die lateral nach innen gerichtete Ausnehmung bildet, und wobei die mittlere Komponente (32) eine ringförmige Rippe (60) umfasst, die den lateral nach außen gerichteten Vorsprung bildet, wobei die Wechselwirkung zwischen der Rippe und der Hinterschneidung bei Verwendung ein Trennen der mittleren von der ersten Komponente in lateraler Richtung verhindert.

14. lmplantat nach einem der vorherigen Ansprüche, wobei die erste Komponente (10) eine phalangiale Komponente eines metatarsophalangialen Gelenkimplantats ist und mit einer Phalanx verbindbar ist, wobei die zweite Komponente (20) eine tarsale Komponente des Gelenks und mit einem Tarsus verbindbar ist.

## Revendications

1. Implant arthroplastique pour constituer une articulation entre un premier et un deuxième os du poignet, de la main ou du pied, l'implant comprenant :
un premier composant (10) définissant une surface concave (14) et comportant un premier moyen de raccordement (18) pour le relier au premier os,
un deuxième composant (20) définissant une surface convexe (24) et comportant un deuxième moyen de raccordement (30) pour le relier au deuxième os,
un composant intermédiaire (32) destiné à être placé entre les premier et deuxième composants et définissant une surface convexe (36) qui peut coulisser sur la surface concave du premier composant pour permettre une articulation entre le premier composant et le composant intermédiaire et une surface concave (34) qui peut coulisser sur la surface convexe du deuxième composant pour permettre une articulation entre le deuxième composant et le composant intermédiaire, et
des moyens (16, 38) pour empêcher le composant intermédiaire de se séparer latéralement d'au moins l'un des premier et deuxième composants.

2. Implant selon la revendication 1, dans lequel la surface concave (14) du premier composant (10) et la surface convexe (36) du composant intermédiaire (32) sont sphériquement incurvées, de façon complémentaire.

3. Implant selon l'une ou l'autre des revendications précédentes, dans lequel la surface convexe (24) du deuxième composant (20) et la surface concave (34) du composant intermédiaire (32) sont définies par les rayons de courbure qui diffèrent dans des directions réciproquement orthogonales.

4. Implant selon l'une quelconque des revendications précédentes, dans lequel la longueur de la surface convexe (24) du deuxième composant (20) dans une direction définie par un rayon de courbure relativement grand est supérieure à la longueur de cette surface dans une direction définie par un rayon de courbure relativement petit.

5. Implant selon l'une quelconque des revendications précédentes, dans lequel les premier et deuxième composants (10, 20) peuvent se déplacer et s'articuler par rapport au composant intermédiaire (32).

6. Implant selon l'une quelconque des revendications précédentes, dans lequel les premier et deuxième composants (10, 20) sont constitués de titane de qualité 5 et leurs surfaces concave et convexe respectives (14, 24) sont dotées d'un revêtement de nitrure de titane.

7. Implant selon l'une quelconque des revendications précédentes, dans lequel le composant intermédiaire (32) est constitué d'une matière plastique à faible frottement.

8. Implant selon la revendication 7, dans lequel la matière plastique est un polyéthylène de poids moléculaire ultra-élevé.

9. Implant selon l'une quelconque des revendications précédentes, dans lequel les premier et deuxième moyens de raccordement (18, 30) comprennent des broches de projection pouvant être situées dans des trous dans les os respectifs.

10. Implant selon l'une quelconque des revendications précédentes et comprenant une projection centrale (16) sur la surface concave (14) du premier composant (10) et une ouverture centrale (38) dans la surface convexe (36) du composant intermédiaire (32), la projection se plaçant lors de l'utilisation, librement dans l'ouverture, pour éviter une séparation latérale du composant intermédiaire et du premier composant.

11. Implant selon la revendication 10, dans lequel la surface concave (14) du premier composant (10) et la surface convexe (36) du composant intermédiaire (32) sont reliées par des arêtes périphériques qui entrent en contact l'une avec l'autre lorsqu'un mouvement relatif entre le premier composant et le composant intermédiaire atteint une limite maximale prédéterminée.

12. Implant selon l'une quelconque des revendications 1 à 9, dans lequel l'un des composants parmi le premier composant (10) et le composant intermédiaire (32) comprend une projection (60) dirigée latéralement vers l'extérieur et l'autre composant parmi le premier composant et le composant intermédiaire comprend une cavité (50) dirigée latéralement vers l'intérieur, la projection interagissant lors de l'utilisation, avec la cavité pour empêcher une séparation latérale du composant intermédiaire et du premier composant.

13. Implant selon la revendication 12, dans lequel le premier composant (10) comprend une paroi annulaire (52) reliant la surface concave (14) de ce composant, la paroi étant formée d'un sillon annulaire (50) définissant la cavité dirigée latéralement vers l'intérieur et le composant intermédiaire (32) comprend une nervure annulaire (60) définissant la projection dirigée latéralement vers l'extérieur, l'interaction, lors de l'utilisation, entre la nervure et le sillon empêchant une séparation latérale du composant intermédiaire et du premier composant.

14. Implant selon l'une quelconque des revendications précédentes, dans lequel le premier composant (10) est un élément de phalange d'un implant articulaire d'une phalange métatarsienne et peut être relié à une phalange, et le deuxième composant (20) est un composant tarsien de l'articulation et peut être relié à un tarse.
